# EUROPEAN PATENT APPLICATION

(11) **EP 4 393 415 A1**
(43) Date of publication of application: **03.07.2024**
(21) Application number: 21954741.1
(22) Date of filing: 12.10.2021
(51) Int. Cl.: A61B 17/12

(54) **WOVEN SINGLE-RIVET DEGRADABLE IMPLANT INSTRUMENT**

(30) Priority: 24.08.2021 CN 202110973290; 24.08.2021 CN 202122006667 U
(71) Applicant: Shanghai Shape Memory Alloy Co., Ltd., Shanghai 201612 (CN)
(72) Inventor: CHEN, Juan, Shanghai 201612 (CN); WANG, Yunbing, Shanghai 201612 (CN); HU, Jinpeng, Shanghai 201612 (CN); WANG, Fan, Shanghai 201612 (CN); ZHANG, Xiang, Shanghai 201612 (CN); LIU, Can, Shanghai 201612 (CN); PAN, Xiangbin, Shanghai 201612 (CN)
(74) Representative: Laufhütte, Dieter
(86) International application number: PCT/CN2021/123173
(87) International publication number: WO 2023/024221

(57) **Abstract**

A woven single-rivet degradable implant instrument, belonging to the technical field of medical instruments. The woven single-rivet degradable implant instrument is provided with a compressed configuration (002) and a free configuration (003) which can be mutually converted, and comprises a first structure (100), a second structure (200), and a middle portion (300) provided between the first structure (100) and the second structure (200), wherein the implant instrument is woven by means of weaving wires made of a degradable material; and the first structure (100) is at least provided with one connecting element (500), the second structure (200) comprises a parabola formed by bending a plurality of wires, and a size-adjustable binding-off opening (250) is formed in one end of the second structure (200) away from the middle portion (300). The provided woven single-rivet degradable implant instrument overcomes the defects in the prior art, and solves the technical problems that in the prior art, due to the use of an occluder made of a metal material, specific complications caused by some metal instruments are prone to occurring, and a degradable wire is relatively soft and difficult to form.

## Description

### TECHNICAL FIELD

The present disclosure relates to a woven single-riveting degradable implant, belonging to the technical field of medical devices. In the present disclosure, the end close to an operator is referred to as a proximal end, and the end far away from the operator is referred to as a distal end.

### BACKGROUND

Occluders used in percutaneous interventional occlusion are mostly made of nitinol, and most of them are double-riveting occluders. However, persistent compression by the nitinol may cause delayed atrioventricular block, and may lead to many potential complications such as long-term metal ion allergy and device-related thrombosis. In addition, patients with implanted metal occluders may not be allowed to undergo ablation of atrial fibrillation, and intervention of mitral valve and left atrial appendage at a later stage. An ideal occluder should act as a bridge to guide a patient's own endothelial tissue to cover the surface of the device, and gradually disappear after the patient's self-repair to avoid long-term complications. Compared with metal materials, biodegradable materials are more flexible and less likely to damage tissues, and thus are an alternative to traditional biocompatible materials and non-biodegradable polymer materials. Degradable polymers such as polylactic acid (PLA), polydioxanone (PDO), polyglycolic acid (PGA), polycaprolactone (PCL) and copolymers thereof have good biocompatibility, which is a hot spot of human tissue engineering research. With the development of materials and processes, biodegradable materials are increasingly used in the field of medical devices, and certain technologies have been applied to implants such as endosseous implants, biodegradable stents and biodegradable occluders.

### SUMMARY

An objective of the present disclosure is to overcome the defects in the prior art to solve the technical problems in the prior art that the occluder made of a metal material may easily cause a variety of potential complications unique to metal devices, such as worn tissues, delayed atrioventricular block, device-related thrombosis, allergic reaction caused by metal ion precipitation, long-term interventional puncture surgery obstacle, and that the degradable thread is relatively soft and difficult to shape.

In order to achieve the objective of solving the above-mentioned problems, the technical solution adopted by the present disclosure is to provide a woven single-riveting degradable implant convertible between a compressed configuration and a free configuration that includes a first structure, a second structure and a middle portion provided between the first structure and the second structure; where the implant is woven with weaving threads made of a degradable material; the first structure is provided with at least one connecting element, and the second structure includes parabolas formed by a plurality of bent threads, each of the parabolas being interlaced with two adjacent parabolas on the left and right to form a plurality of intersections, and having an apex oriented toward one end of the second structure away from the middle portion on a circumstance of a virtual circle so that said each parabola is tangent to the virtual circle at the apex.

Preferably, the second structure is provided with an opening at the one end of the second structure away from the middle portion.

Preferably, the opening in the second structure is a size-adjustable contractile opening, which is formed by passing at least one thread sequentially through arcs where respective apexes of the parabolas are located in the second structure or through the plurality of intersections.

Preferably, the size-adjustable contractile opening has a diameter set between 0 and 30 mm.

Preferably, the size-adjustable contractile opening as well as cross-sections of the first structure, the second structure and the middle portion are centered on the same central axis.

Preferably, any one of the first structure, the second structure and the middle portion is provided with at least one radiopaque marker; the connecting element is configured to be connected to a delivery device for the implant, and is formed by bundling the degradable thread material; the implant is provided with at least one auxiliary forming thread passing through any surface of the implant; and the implant is woven in such a way that M threads are pressed against N threads.

Preferably, the implant has a surface provided with a layer of a drug and/or a hydrophobic material and/or an anticoagulant drug for inhibiting endothelialization.

Preferably, the implant has a surface provided with a layer of a drug and/or a hydrophobic material and/or an anticoagulant drug for promoting endothelialization.

The present disclosure provides a weaving tool for weaving the woven single-riveting degradable implant as described above, which includes a hollow weaving tool shaped in a cylinder open at both ends, where protruding linear ribs are evenly provided on an outer wall of the cylinder, the ribs extending parallel to a central axis of the cylinder beyond both ends of the cylinder.

The present disclosure provides a method of weaving the woven single-riveting degradable implant as described above, which includes:
Step 1 of providing the above-mentioned weaving tool shaped in a cylinder with a plurality of ribs provided on an outer wall of the weaving tool, the weaving tool being coaxial with the second structure; hanging around the plurality of ribs respective parabolas formed by bending at least one thread to form arcs oriented towards a center of an end of the second structure, each parabola being interlaced with adjacent parabolas to form a plurality of intersections, and forming a mesh structure by further weaving;
Step 2 of performing shape-setting by heat treatment on the mesh structure obtained in the step 1, and forming a size-adjustable contractile opening by passing a weaving thread at one end of the mesh structure or another thread through arcs where respective apexes of the parabolas at the one end of the mesh structure are located or through intersections near the one end;
Step 3 of thermally fusing head portions of all the weaving threads at the other end of the mesh structure to form a connecting element; and
Step 4 of placing a specific mold into the fused mesh structure and then performing shape-setting by heat treatment on the mesh structure to finally produce the woven single-riveting degradable implant.

Compared to the prior art, the present disclosure has the following advantageous effects. The present disclosure provides a woven single-riveting degradable implant, which can avoid the problems of complications unique to metal devices, such as worn tissues, delayed atrioventricular block, device-related thrombosis, allergic reaction caused by metal ion precipitation, and long-term interventional puncture surgery obstacle. The present disclosure is applicable to atrial septal defect occluders, ventricular septal defect occluders, patent ductus arteriosus occluders, patent foramen ovale occluders, atrial shunts, vascular plugs and left atrial appendage occluders, paravalvular leak occluders, and the like. The present disclosure overcomes the defects of the prior art to solve the technical problems of the prior art that the occluder made of a metallic material easily causes some complications unique to metallic devices and that the degradable thread is relatively soft so as to be difficult to shape.

### BRIEF DESCRIPTION OF THE DRAWINGS

FIG. 1 is a schematic structural diagram showing a compressed configuration according to the present disclosure.
FIG. 2 is a schematic diagram showing a free configuration of a first embodiment according to the present disclosure.
FIG. 3 is a bottom view of a first embodiment according to the present disclosure.
FIG. 4 is a schematic diagram showing the free configuration of a second example according to the present disclosure.
FIG. 5 is a top view of a second structure according to the present disclosure.
FIG. 6 is a partial enlarged view of a second structure according to the present disclosure.
FIG. 7 is a partial enlarged view of FIG. 6.
FIG. 8 is a first schematic diagram showing a weaving tool according to the present disclosure.
FIG. 9 is a second schematic diagram showing a weaving tool according to the present disclosure.
FIG. 10 is a top view of a weaving tool according to the present disclosure, i.e., a schematic diagram showing ribs.
FIG. 11 is a schematic diagram showing a winding pattern of weaving threads using ribs according to the present disclosure.
FIG. 12 is a partial enlarged view of FIG. 10.
FIG. 13 is a mesh woven structure after shape-setting by heat treatment according to the present disclosure.

### DETAILED DESCRIPTION

In order to make the present disclosure more apparent for better understanding, preferred embodiments thereof are now described in detail with reference to the accompanying drawings as follows.

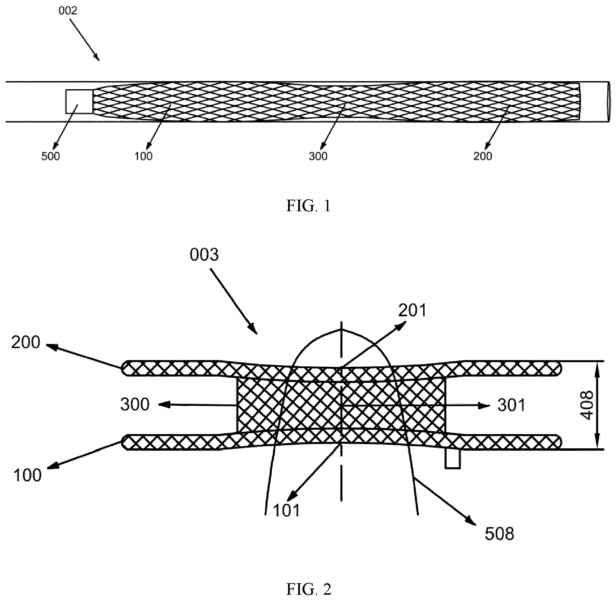

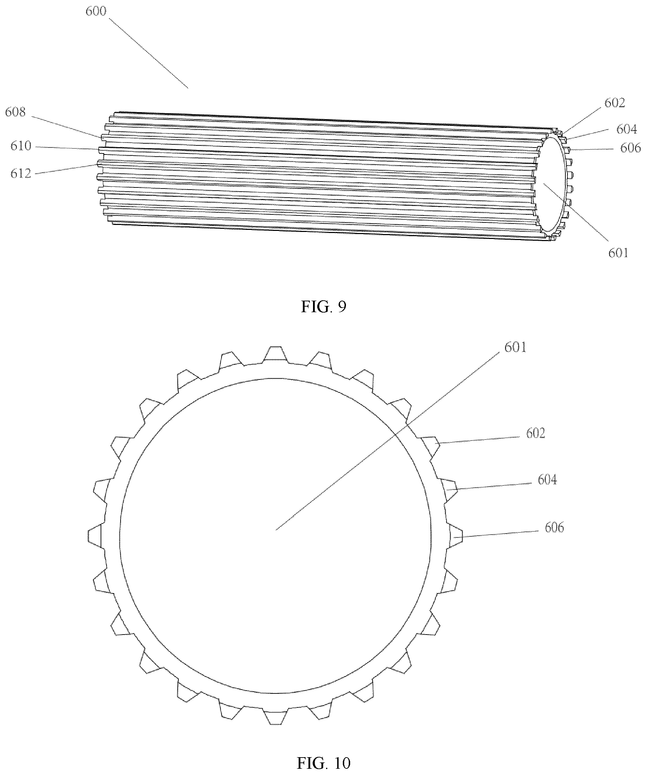

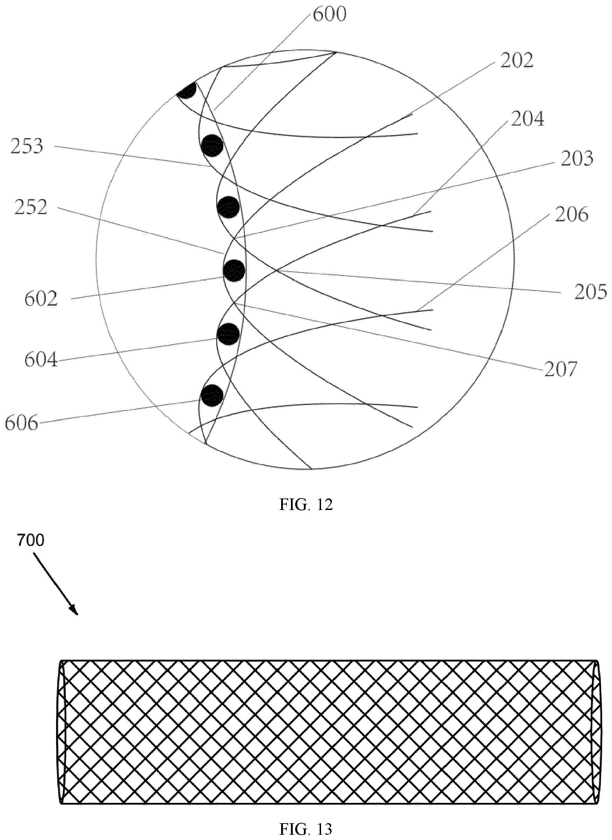

As shown in FIGS. 1-13, the present disclosure provides a woven single-riveting degradable implant convertible between a compressed configuration 002 and a free configuration 003, including a first structure 100, a second structure 200, and a middle portion 300 provided between the first structure 100 and the second structure 200; where the implant is woven from weaving threads made of a degradable material; the first structure 100 is provided with at least one connecting element 500, and the second structure 200 includes parabolas formed by a plurality of bent threads, each parabola being interlaced with two adjacent parabolas on the left and right to form a plurality of intersections, and having an apex oriented toward one end of the second structure away from the middle portion 300 and disposed on a circumstance of a virtual circle so that said each parabola is tangent to the virtual circle at the apex. The second structure 200 is provided with an opening at the one end of the second structure 200 away from the middle portion 300. The opening in the second structure may be provided as a size-adjustable contractile opening 250, which is formed by passing at least one thread 240 sequentially through arcs where respective apexes of the parabolas are located in the second structure 200 or through the plurality of intersections. The size-adjustable contractile opening 250 may have a diameter between 0 and 30 mm. The size-adjustable contractile opening 250 as well as cross-sections of the first structure, the second structure and the middle portion are centered on the same central axis. Any one of the first structure 100, the second structure 200 and the middle portion 300 may be provided with at least one radiopaque marker; the connecting element 500 may be configured to be connected to a delivery device for the implant, and is formed by bundling the degradable thread material; the implant may be provided with at least one auxiliary forming thread 508 passing through any surface of the implant; and the implant is woven in such a way that M threads are pressed against N threads. The implant may have a surface provided with a layer of a drug and/or a hydrophobic material and/or an anticoagulant drug for inhibiting endothelialization. The implant may have a surface provided with a layer of a drug and/or a hydrophobic material and/or an anticoagulant drug for promoting endothelialization.

The present disclosure provides a weaving tool for weaving the woven the woven single-riveting degradable implant as described above, which includes a hollow weaving tool 600 shaped in a cylinder open at both ends, wherein protruding linear ribs are evenly provided on an outer wall of the cylinder, the ribs extending parallel to the central axis of the cylinder beyond both ends of the cylinder.

The present disclosure provides a method of weaving a woven single-riveting degradable implant as described above, which includes:
Step 1 of providing the above-mentioned weaving tool 600 shaped in a cylinder with a plurality of ribs provided on an outer wall of the weaving tool 600, the weaving tool 600 being coaxial with the second structure 200; hanging around the plurality of rib respective parabolas formed by bending at least one thread to form arcs oriented toward a center of an end of the second structure, each parabola being interlaced with adjacent parabolas to form a plurality of intersections, and forming a mesh structure 700 by further weaving;
Step 2 of performing shape-setting by heat treatment on the mesh structure 700 obtained in step 1, and forming a size-adjustable contractile opening 250 by passing a weaving thread at one end of the mesh structure or another thread through arcs where respective apexes of the parabolas at the one end of the mesh structure are located or through intersections near the one end;
Step 3 of thermally fusing head portions of all the weaving threads at the other end of the mesh structure to form a connecting element 500; and
Step 4 of placing a specific mold into the fused mesh structure, to finally obtain the woven single-riveting degradable implant.

### Examples

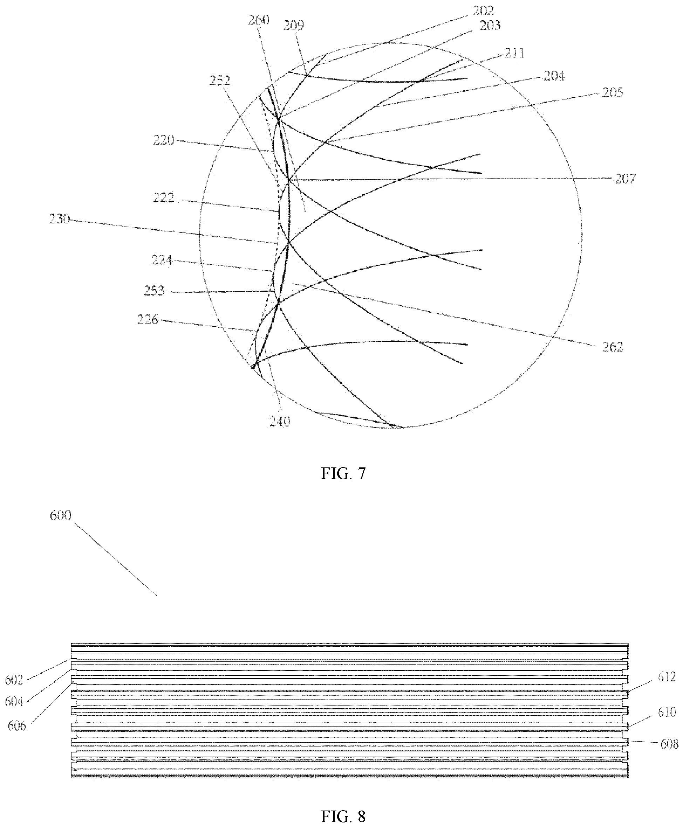

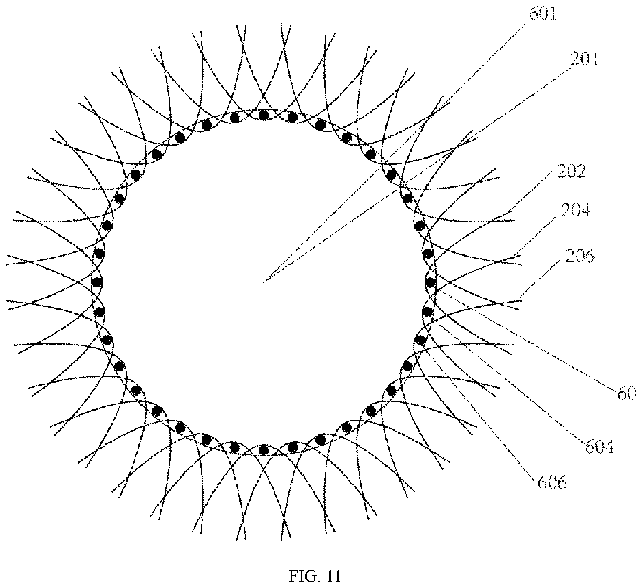

A weaving tool is selected, such as the weaving tool shown in FIGS. 8, 9 and 10. A cylindrical weaving tool 600 having several ribs 602, 604, 606, 608, 610 and 612 on outer edges of both ends is used, where weaving threads are hung around ribs 602, 604 and 606 to be interlaced and woven to form a mesh structure 700, as shown in FIGS. 11, 12 and 13.

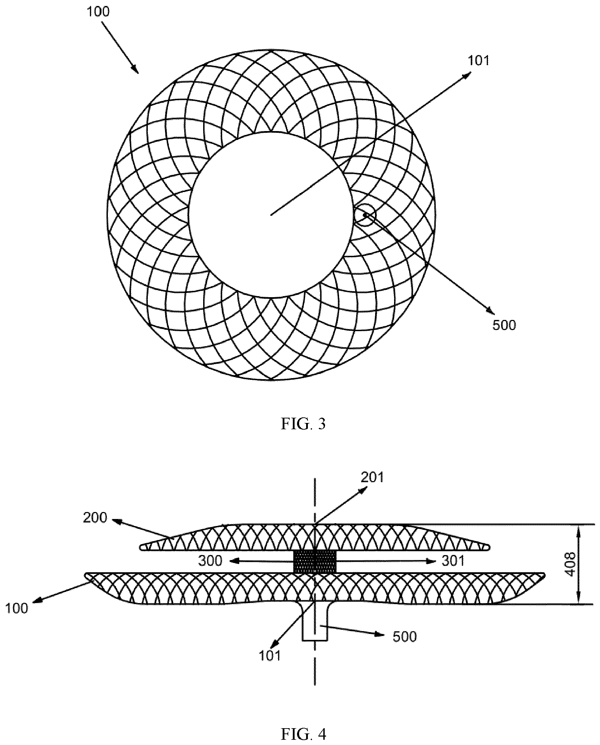

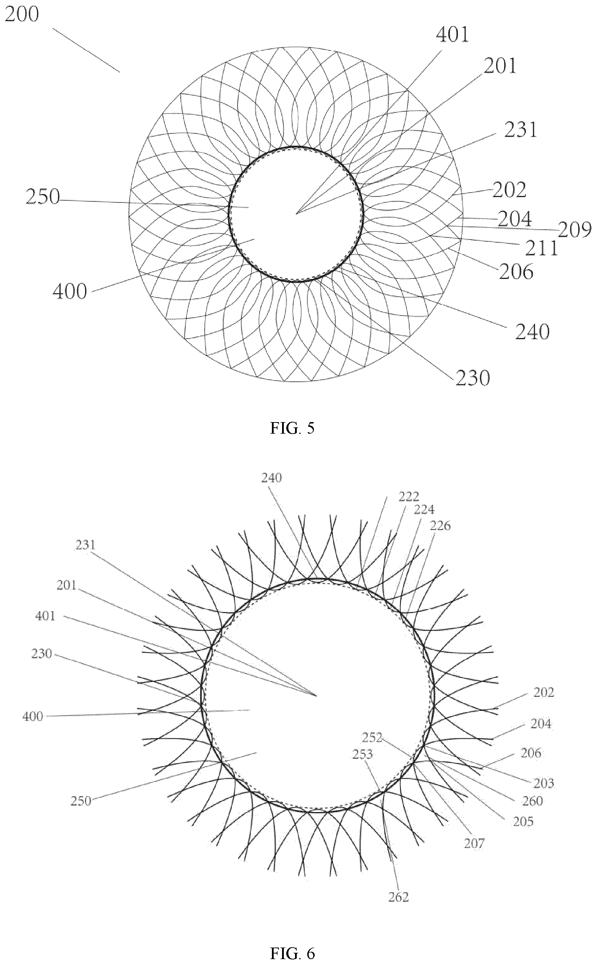

The mesh structure 700 is subjected to shape-setting by heat treatment, and then one end of the weaving thread or another thread 240 is passed through each arc 252, 253 at one end of the mesh structure or passed through all the intersections 203, 205 closest to the center of circle 201 to form a size-adjustable contractile opening 250, where the size-adjustable contractile opening 250 has a size of 0-30 mm, as shown in FIGS. 5, 6 and 7; the head portions of the weaving threads at the other end are all thermally fused to form a connecting element 500; then a specific mold is placed into the mesh structure 700 processed above, thereby producing a woven single-riveting degradable implant. FIGS. 2 and 4 respectively a woven single-riveting degradable atrial shunt and a woven single-riveting degradable patent foramen ovale occlude. When the size of the size-adjustable contractile opening 250 is approximately 0, the size of the size-adjustable contractile opening 250 of the second structure 200 is equal to or approximately 0, and the second structure is shaped in a closed disc, which can be suitable for occlusion; when 0 <the size of the size-adjustable contractile opening 250 ≤ 30mm, the size-adjustable contractile opening 250 is sized to match the through-hole (i.e., shunt hole) of the atrial shunt, and the through-hole penetrates the entire instrument in the axial direction to form a shunt hole.

Example 1: FIGS. 2 and 3 show a woven single-riveting degradable atrial shunt provided in the present disclosure. FIG. 5 is a top view of the second structure, where the size of the through-hole 400 is the size of the size-adjustable contractile opening 250, i.e., the size of the through-hole 400 (i.e., the shunt hole) for allowing blood in the left atrium to flow through the through-hole 400 into the right atrium to relieve the pressure in the left atrium. In addition, the location of the connecting element 500 is offset from the center point due to the presence of the shunt hole.

Example 2: FIG. 4 shows a woven single-riveting degradable patent foramen ovale occluder according to the present disclosure, where the size of the through hole 400 is close to zero by contracting the size-adjustable contractile opening 250, i.e., there is no shunt hole, and the connecting element 500 is located at the center of the first structure 100.

The present disclosure provides a woven degradable implant device having a compressed configuration 002 and a free configuration 003, the device including a first structure 100, a second structure 200 and a middle portion 300 located between the first structure and the second structure.

The first structure has at least one connecting element 500.

The device as a whole is woven by at least one weaving thread of a degradable material.

The second structure 200 includes parabolas 202, 204, 206 formed by bending at least A (4≤A≤144) threads, where each parabola, such as the parabola 204, is interlaced with parabolas 202, 206 on the left and right to form several intersections 203, 205, 207, 209, 211, and in the direction extending toward the center of circle 201, at each three intersections 203, 205, 207 closest to the center of circle 201, parabolas 202, 204, 206 are cut by each other to form a plurality of fan-shaped closed loops 260, 262, respective apexes 220, 222, 224, 226 of the parabolas may form a virtual circle 230 to which the parabolas are tangent at the respective apexes 220, 222, 224, 226, the center 231 of the virtual circle 230 corresponding to the center 201 of the end cross-section of the second structure. A through hole 400 is located at the end of the second structure 200, and the center 401 of the through hole corresponds to the center 201 of the cross section of the end of the second structure 200 and corresponds to the center 231 of the virtual circle 230; the arcs 252, 253 of all the fan-shaped closed loops 260, 262 are connected to form the through hole 400; at least one thread 240 in the second structure 200 passes through the arcs 252, 253 of all the fan-shaped closed loops 260, 262 in sequence to form a size-adjustable contractile opening 250; the size of the size-adjustable contractile opening 250 corresponds to the size of the through-hole 400; the through hole 400 has a diameter between 0 mm and 30 mm, and a height 408 between 2 mm and 30 mm; the center 401 of the cross section of the through hole 400 is on the same axis as the centers 101, 201, 301 of the cross sections of the first structure 100, the second structure 200 and the middle portion 300; the cross-sectional areas of the through-hole 400 and the size-adjustable contractile opening 250 is approximately equal to the cross-sectional area of the virtual circle 230; the first structure 100 refers to the end portion near the operator and the second structure 200 refers to the end portion away from the operator. Any part of the first structure 100, the second structure 200 and the middle portion 300 has at least one radiopaque marker; the connecting element 500 is to be connected to the delivery device, and the connecting element 500 can be formed by bundling the degradable thread material and/or other degradable material at the proximal end of the device; the connecting element 500 is to be connected to the delivery device, and the connecting element 500 may be a degradable thread material clamped at the proximal end by a fixator; there may be at least one auxiliary forming thread 508 passing through any surface of the device.

The device may be woven by pressing M threads (M ≥ 1) across N threads (N ≥ 1).

The surface of the device may be coated with a layer of a drug and/or a hydrophobic material and/or an anticoagulant drug for inhibiting endothelialization.

The surface of the device may be coated with a layer of a drug and/or a hydrophobic material and/or an anticoagulant drug for promoting endothelialization.

The compressed configuration 002 is of an elongated strip shape, and the free configuration 003 is a normal working state of the device.

A method of weaving a degradable implant as a method for preparing the device includes the following operations.

A cylindrical weaving tool 600 having several ribs 602, 604, 606, 608, 610, 612 on outer edges of both ends is used, where the center 601 of the cross-section at the end of the weaving tool 600 is on the same axis as the center 201 of the cross-section at the end of the second structure 200. Parabolas 202, 204, 206 formed by bending at least one thread are hung around ribs 602, 604, 606 respectively to form arcs 252, 253 closest to the center 201, each parabola 204 being interlaced with adjacent parabolas 202, 206 to form several intersections 203, 205, 207, 209, 211 to form a mesh structure 700.

The mesh structure 700 is subjected to shape-setting by heat treatment, then one end of the weaving thread or another thread 240 is passed through each arc 252, 253 at one end of the mesh structure or passed through all the intersections 203, 205 closest to the center of circle 201 to form a size-adjustable contractile opening 250, and head portions of all the weaving threads at the other end are thermally fused to form a connecting element 500. A specific mold is placed into the fused mesh structure, which is then subjected to shape-setting by heat treatment again, thereby producing a woven single-riveting degradable implant.

The above are only preferred embodiments of the present disclosure, and do not limit the present invention in any form or substance. It should be understood that those of ordinary skill in the art can also make several improvements and additions without departing from the present disclosure, and these improvements and additions should also be regarded as being included in the protection scope of the present disclosure. When those skilled in the art who are familiar with the art can make slight changes, modifications and equivalent changes based on the technical content disclosed above without departing from the spirit and scope of the invention, the resulting embodiments are all equivalent embodiments; at the same time, any equivalent changes, modifications and evolutions made to the above embodiments based on the essential technology of the present invention still fall within the scope of the technical solution of the present disclosure.

## Claims

1. A woven single-riveting degradable implant convertible between a compressed configuration and a free configuration, comprising a first structure, a second structure, and a middle portion provided between the first structure and the second structure, wherein the implant is woven with weaving threads made of a degradable material; the first structure is provided with at least one connecting element, and the second structure comprises parabolas formed by a plurality of bent threads, each parabola being interlaced with two adjacent parabolas on the left and right to form a plurality of intersections, and having an apex oriented toward one end of the second structure away from the middle portion and disposed on a circumstance of a virtual circle, so that said each parabola is tangent to the virtual circle at the apex.

2. The woven single-riveting degradable implant according to claim 1, wherein the second structure is provided with an opening at the one end of the second structure away from the middle portion.

3. The woven single-riveting degradable implant according to claim 2, wherein the opening in the second structure is a size-adjustable contractile opening, which is formed by passing at least one thread sequentially through arcs where respective apexes of the parabolas are located in the second structure or through the plurality of intersections.

4. The woven single-riveting degradable implant according to claim 3, wherein the size-adjustable contractile opening has a diameter of 0-30 mm.

5. The woven single-riveting degradable implant according to claim 4, wherein the size-adjustable contractile opening as well as cross-sections of the first structure, the second structure and the middle portion are centered on the same central axis.

6. The woven single-riveting degradable implant according to claim 5, wherein any one of the first structure, the second structure and the middle portion is provided with at least one radiopaque marker; the connecting element is configured to be connected to a delivery device for the implant, and is formed by bundling the degradable thread material; the implant is provided with at least one auxiliary forming thread passing through any surface of the implant; and the implant is woven in such a way that M threads are pressed against N threads.

7. The woven single-riveting degradable implant according to claim 6, wherein the implant has a surface provided with a layer of a drug and/or a hydrophobic material and/or an anticoagulant drug for inhibiting endothelialization.

8. The woven single-riveting degradable implant according to claim 6, wherein the implant has a surface provided with a layer of a drug and/or a hydrophobic material and/or an anticoagulant drug for promoting endothelialization.

9. A weaving tool for weaving a woven single-riveting degradable implant according to any one of claims 1 to 8, comprising a hollow weaving tool shaped in a cylinder open at both ends, wherein protruding linear ribs are evenly provided on an outer wall of the cylinder, the ribs extending parallel to a central axis of the cylinder beyond both ends of the cylinder.

10. A method of weaving a woven single-riveting degradable implant according to any one of claims 1 to 8, comprising:
Step 1 of providing a weaving tool according to claim 9 shaped in a cylinder with a plurality of ribs provided on an outer wall of the weaving tool, the weaving tool being coaxial with the second structure; hanging around the plurality of ribs respective parabolas formed by bending at least one thread to form arcs oriented toward a center of an end of the second structure, each parabola being interlaced with adjacent parabolas to form a plurality of intersections, and forming a mesh structure by further weaving;
Step 2 of performing shape-setting by heat treatment on the mesh structure obtained in the step 1, and forming a size-adjustable contractile opening by passing a weaving thread at one end of the mesh structure or another thread through arcs where respective apexes of the parabolas at the one end of the mesh structure are located or through the intersections close to the one end;
Step 3 of thermally fusing head portions of all the weaving threads at the other end of the mesh structure to form a connecting element; and
Step 4 of placing a specific mold into the fused mesh structure, and performing shape-setting by heat treatment on the mesh structure to finally produce the woven single-riveting degradable implant.
